# EUROPEAN PATENT SPECIFICATION

(11) **EP 0 654 287 B1**
(45) Date of publication and mention of the grant of the patent: **05.08.1998**
(21) Application number: 94306403.0
(22) Date of filing: 31.08.1994
(51) Int. Cl.: A63B 23/035, A61B 17/132

(54) **Tightening strap for muscle training**
Spannriemen zum Muskeltraining
Dispositif de tension pour exercices musculaires

(30) Priority: 22.11.1993 JP 313949/93
(43) Date of publication of application: 24.05.1995
(73) Proprietor: Best Life Co., Ltd., Fuchu-shi, Tokyo (JP)
(72) Inventor: Sato, Yoshiaki, Fuchu-Shi, Tokyo (JP)
(74) Representative: Jackson, Peter Arthur

(56) References cited:
- EP-A- 0 408 049
- WO-A-92/05741
- US-A- 4 273 328
- US-A- 5 015 251

## Description

The present invention relates to an accessory tool to be used for muscle building and also to a muscle building method using the accessory tool.

In muscle building, muscular strength and/or size is generally increased by applying a load to a desired muscle or group of muscles using a weight such as a dumbbell or barbell, or using resistance based on elasticity, for example, of a spring, rubber, or other resilient material, and by carrying out exercises to stretch and contract the desired muscle in such a loaded condition to such a degree as to obtain a given fatigue. Using this type of exercise, the muscle building effect is improved only by increasing the weight or the resistance of the training tool or by increasing the number of exercises carried out. However, even when the load to the desired muscle is unduly increased, an increased quantity of the load is dispersed to other muscles. As a result, the other muscles are undesirably strengthened, and in some cases, the muscles or joints may be damaged.

Many studies on the effect of exercise on the muscles have been made including studies of the determination of causes of muscle fatigue, muscular growth and the relationship between muscles and load. As a result of these studies, it is known that muscles are fatigued as soon as the flow of blood is restricted, that isometric muscle contraction caused by lifting or supporting a heavy object is more effective for enlargement of the muscles than isotonic muscle contraction caused by running or swimming, and that muscular strength is more effectively developed by applying a load at a given level which can be increased. In a publication from 1967, it was reported that exercise with the flow of blood restricted remarkably enhances muscular strength though a fundamental mechanism thereof has not yet been clarified [see "Sports Medicine", ed. Eiichiro Hisamatsu and Michio Ikai, (K.K. Kyorin Shoin and Taiiku-no-kagakusha, September 20, 1967) 4th ed., pp 203 and 206].

The present inventor has long been concerned with the study of muscle building to search for an effective training tool and a training method using it. However, the current thinking regarding sports and training is that it is important that the flow of blood to the muscles should be efficient and should be accelerated to supply oxygen to the muscles and thereby prevent build up of lactic acid which causes fatigue. Accordingly, the publication by Hisamatsu and Ikai referenced above is directly opposed to the presently accepted understanding of sports training. Further, there is medical concern that restricting the flow of blood may be harmful to a living body.

Conventional muscle building is carried out as an auxiliary training exercise, separate from the sport for which the training is required. As far as the present inventor is aware, there exists at present, no exercise which results in muscle building directly adapted for and which can be combined with carrying out a specific sport.

The present inventor has examined the effect of restricting the blood flow to a muscle (as used herein "muscle" refers to one muscle or a group of muscles) to ascertain its effectiveness as a muscle building method useful for general sports, and also has examined training tools suitable for use in muscle building.

The present invention aims to provide a training tool and a training method which will improve training efficiency, so that a desired muscle can be strengthened and which is directly adapted for a specific sport because the muscle building is effected as the specific sport is being carried out.

In accordance with one aspect of the present invention, there is provided a tightening tool for sports training comprising an elastic member which is arranged to form a loop of variable circumference for encircling a selected part of a limb, and locking means for holding the loop formed from the flexible member during use at a desired circumference; characterised in that the tool comprises at least a detecting portion of indicating means for detecting the force exerted by the loop on the encircled part of a limb, in use.

The invention also includes a method for enlarging a muscle of a human being, the method comprising encircling a selected part of a limb with a loop of the new tightening tool, and reducing the circumference of the loop to tighten the loop thereby restricting flow of blood to the muscle to be enlarged. Preferably, the reduced circumference of the loop is maintained while the muscle is exercised. The exercise may involve contracting the muscle isometrically or isotonically.

Thus the tightening tool according to the present invention is designed to form an adjustable tightening loop. The tightening loop is used to encircle an arm or leg and the circumference reduced so that it can be tightened around a selected part of the lim. For example, the tool may be positioned so that the loop encircles an arm near a shoulder joint. On tightening, the blood flow to the arm is restricted. Exercises for the arm muscles are then performed using a dumbbell, for example, maintaining the tightened position of the tightening tool. As a result, the training effect obtained using a light dumbbell is comparable to that using a heavy dumbbell without the use of the tightening tool of the invention. Furthermore, training time can be shortened and in addition, adverse effects to muscles which do not require exercise and also damage to joints can be effectively avoided.

The present invention therefore provides that muscle building can be carried out simultaneously with playing or carrying out the desired sport. In a bicycle sport, for example, the muscles can be strengthened during pedalling a bicycle. Further, in any other sports such as rugby, football, skiing, skating, swimming, and sumo, the muscles can be strengthened during play or whilst a particular form of exercise is carried out, and any special training prepared for the intended sport can be omitted. Accordingly, the training tool and the training method according to this invention provide a significant benefit over prior art training methods.

The invention may be used for the enlargement of a muscle of any mammal, however generally the subject is a human being. Preferably, the method of the invention will be carried out while the subject carries out a sporting activity, so that muscular contractions will be during play or whilst carrying out a sport such as skiing, ice skating, sumo wrestling or cycling. In this way a healthy muscle can be enlarged.

In the method of the invention in which the muscle to be enlarged is contracted, preferably the reduced circumference of the loop is maintained for no greater than 30 minutes, most preferably no greater than 15 minutes.

The method of the present invention is particularly directed to methods of muscle enlargement of a subject, for improving the sporting prowess of that subject. The method of the invention is also directed to methods of muscle enlargement to improve the aesthetic appearance of a subject. It will be appreciated from the above that the methods of the invention do not include methods of muscle enlargement which are methods of medical treatment.

While the cause of an increase in the training effect of exercise in combination with the use of the tightening tool of this invention is not fully understood, without wishing to be bound by theory, the following mechanism is proposed.

It is well known that muscular strength is increased by so-called "super-recovery", whereby muscles which are fatigued through training recover from fatigue, and the condition of the recovered muscles exceeds the initial condition. Accordingly, training efficiency can be increased by providing training conditions which will efficiently generate muscle fatigue.

Muscle fatigue is largely related to the supply of energy and oxygen to the muscles and the treatment of lactic acid generated in the course of energy metabolism. These conditions are largely dependent upon the flow of blood to the muscles. Accordingly, fatigue can be efficiently generated in a desired muscle by restricting the flow of blood to the desired muscle using the tightening tool of this invention.

As the tightening tool is provided with an indicating means for indicating a tightening force imposed on the selected part of the limb, such an indicating means will usually confirm when an optimum tightening force has been exerted by the tightening tool, and the same tightening force can be easily reproduced at all times.

In a further embodiment of the invention, the tightening tool additionally comprises a lining, in use the lining being innermost along the circumference of the loop formed from the flexible member. The lining will therefore be positioned beneath the flexible member, at the surface of the tightening tool contacting the limb of a subject so as to protect the skin and absorb sweat. The lining is preferably formed of a material having softness, breathability, and moisture absorbency.

Further, it is to be noted that the term "muscular strength" used in the above and following description has a meaning equivalent to that of " " muscle or muscles".

In the accompanying drawings,
Fig. 1 is a perspective view of a tightening tool according to a first construction which is not part of the present invention;
Fig. 2 is a perspective view of a tightening tool according to a second construction which is not part of the present invention; and,
Fig. 3 is a perspective view of a tightening tool according to a preferred embodiment of the present invention.

In the following description and the drawings, common parts in the different views will be denoted by the same reference numerals and double description thereof will be omitted as required.

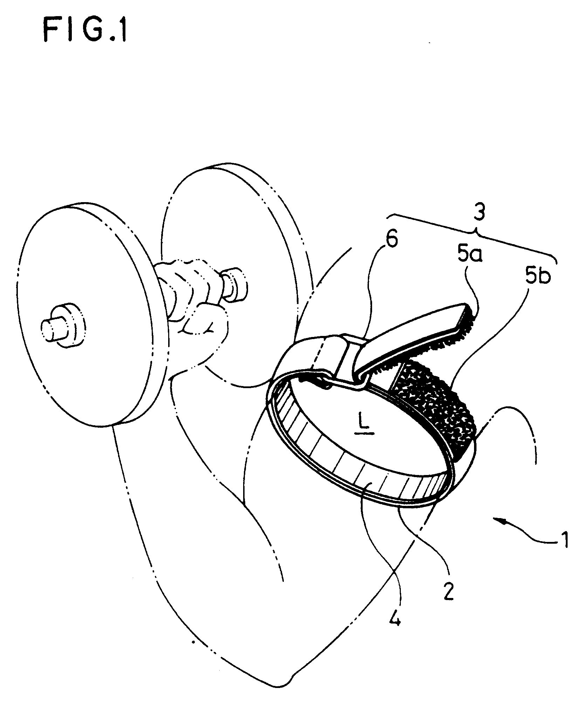

In Fig. 1, reference numeral 1 generally denotes a tightening tool according to the said first construction. The tightening tool 1 is composed of a body 2 and a locking means 3. The body 2 is formed as a belt of an elastic material such as rubber. The body 2 is provided with a lining 4 adapted to come into direct contact with a human skin. The lining 4 is formed of a material having a high elasticity and a high water absorbency.

The locking means 3 is composed of a pair of first and second fastener strips 5a and 5b formed at one end portion of the body 2 and a rectangular holding ring 6 attached to the other end portion of the body 2 by sewing. The tightening tool 1 thus constructed is used in the following manner. The portion of the body 2 at which the first fastener strip 5a is formed is passed through the holding ring 6. Then, the body 2 is folded at a suitable portion to bring the first fastener strip 5a into opposed relationship to the second fastener strip 5b. In this condition, the first fastener strip 5a is pressed on the second fastener strip 5b to be fastened together. As a result, a tightening loop L with a desired diameter is formed inside the lining 4 of the body 2 so as to surround a desired part of a human body. Thus, the desired part can be tightened by the tightening tool 1 with desired tightening force, and such a tightened condition can be maintained.

While Fig. 1 shows a condition that a part between a deltoid muscle and a biceps muscle of arm of the human body is tightened by the tightening tool 1, this condition is merely illustrative and the tightening tool 1 may be, of course, applied to various other parts of the human body. Further, the size of the tightening tool 1 is not limited. Furthermore, some tightening tools 1 may be suitably combined in such a way that they are crossed to be applied to both shoulders of the human body. In addition, the body 2 may be suitably designed in color, pattern, shape, etc. to obtain a good appearance fit for the scene of sports or training.

To confirm the effect of the tightening tool 1 mentioned above, training was subjected to a group of persons using the tightening tool 1 and another group of persons not using the tightening tool 1. The training was carried out by each person for an hour a day and was repeated twice a week for six months. Such an hour training was comprised of barbell curl and reverse push up. The tightening tool 1 was applied between the deltoid muscle and the biceps muscle of arm. The circumferential size of the biceps muscle of arm was measured before and after the six-month training to confirm the result : the muscular strengthening effect on the circumferential sizes in the group using the tightening tool 1 was about three times those in the group not using the tightening tool 1. This result proves that the tightening tool 1 is effective for strengthening of the muscle.

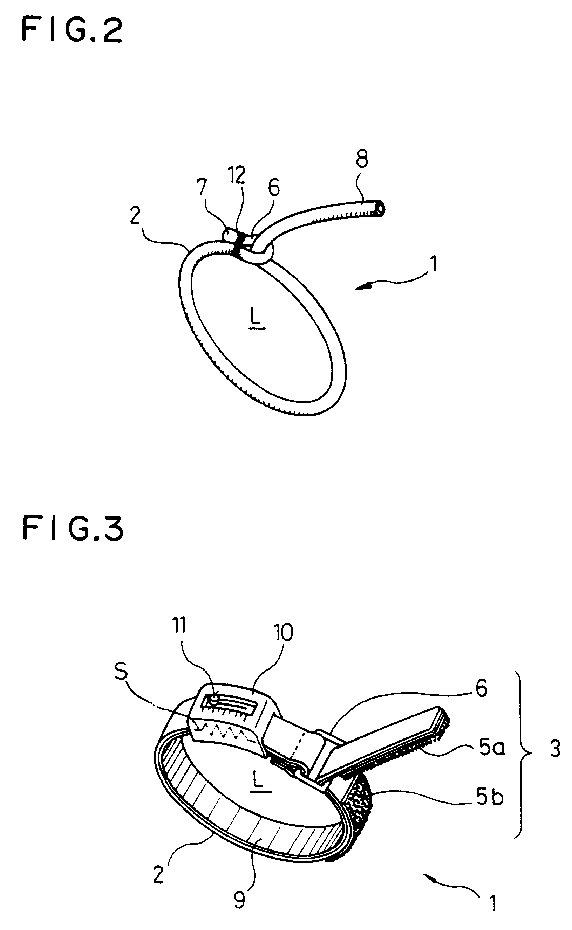

In the Fig. 2 construction, a hollow cylindrical, rubber tube having a suitable thickness is used as the body 2 to provide a simple structure. One end portion 7 of the body 2 is turned back to be fixed by winding a string 12 around the body 2, thus forming a holding ring 6 as a locking means. The holding ring 6 has an inner diameter somewhat smaller than an outer diameter of the rubber tube. The other end portion 8 of the body 2 is forcibly inserted through the holding ring 6 to thereby form a tightening loop L inside the body 2. In operation, the other end portion 8 inserted through the holding ring 6 is suitably drawn out to thereby elastically deform the holding ring 6 and a part of the other end portion 8 contacting with the inside surface of the holding ring 6. Accordingly, a contact area and a contact strength between the holding ring 6 and this part of the other end portion 8 are increased to obtain a large frictional force exerted between the rubber surfaces. As a result, the tightening loop L can be maintained at a desired size.

In the preferred embodiment according to Fig. 3, an indicating means 10 for indicating tightening force is connected to the body 2, and the structure of the locking means 3 is basically similar to that in the Fig. 1 construction. The body 2 is formed as a belt of an inextensible material such as a cloth belt, and a nonwoven fabric 9 as a lining is attached to the inside surface of the body 2 to protect the human skin and provide a feel of comfortable touch. In this preferred embodiment, a spring S is used in the indicating means 10, and the body 2 is separated into two parts at the indicating means 10, wherein opposed ends (not shown) of the two separate parts of the body 2 are connected to both ends of the spring S. The quantity of deformation of the spring S is indicated as a displacement of a pointer 11 connected to the spring S. As another embodiment of the indicating means 10, means for electrically detecting tightening force and indicating a result of detection may be used. In this case, only a detecting portion for detecting the tightening force may be provided on the body 2, and an indicating portion may be connected through a connecting cord to the detecting portion and be provided separately from the body 2, thereby lightening the weight of the tightening tool 1. As still another embodiment of the indicating means 10, any mark such as a scale may be provided on the outside surface of the body 2, thereby further lightening the weight and simplifying the structure.

As described above, the tightening tool for muscle training according to the present invention functions to moderately block the flow of blood to a target muscle to be strengthened and thereby efficiently generate fatigue in the target muscle. Accordingly, by carrying out the muscle training in this condition, a training time can be reduced, and simultaneously any troubles such as an adverse effect to other unintended muscles and a damage to joints can be effectively avoided.

Further, the tightening tool according to the present invention includes the locking means capable of locking the tightening loop at a desired size by a simple operation. Accordingly, a proper tightening force can be desirably obtained, and a troublesome operation for tightening can be eliminated.

Further, when the tightening tool is equipped with the indicating means for indicating a tightening force, the tightening force can be easily monitored by sight, and proper tightening force can be easily reproduced at all times.

Further, when the lining for protecting the human skin is attached to the inside surface of the body of the tightening tool, the human skin can be protected from damage, and a feel of comfortable touch can be obtained.

### Example I

The tightening tool as shown in Fig. 1 was used to carry out training for strengthening the muscles of an upper arm and a thigh.

The body of the tightening tool used for the upper arm was formed as an elastic neoprene belt having a width of 25 mm and a thickness of 3 mm, and the body of the tightening tool used for the thigh was formed as an elastic neoprene belt having a width of 38 mm and a thickness of 3 mm. A Velcro fastener was attached to each body to form the locking means, so as to easily form the tightening loop by a simple operation.

Fifty men aged 19 to 45 were selected as a subject for muscle training, and the tightening tools were applied to a thinner one of a pair of right and left upper arms of each man and a thinner one of a pair of right and left thighs of each man. The reason why the thinner upper arm and the thinner thigh were selected is that they are weaker in muscular strength than the thicker upper arm and the thicker thigh, respectively.

In more detail, the tightening tool for the upper arm was applied between a deltoid muscle and a biceps muscle of arm of each man, and the tightening tool for the thigh was applied between a quadriceps muscle of thigh/a biceps muscle of thigh and a greatest gluteal muscle of each man. The subject parts of the muscles were tightened by the tightening tools for about 30 minutes to block the flow of blood. Since the conditions of the muscles of all the men are different, of course, the tightening time was set especially in consideration of arm and thigh thicknesses, condition of fat put on, age, muscle thickness depending upon experience of exercise, condition of health (any men other than one prohibited from doing sports by a doctor was permitted to carry out this training), etc. In particular, in the event that the fingers of the hand or the foot here numbed to lose sensation, the tightened condition was immediately released to end the muscle training.

A degree of tightening was determined so that a condition where the blood vessel in the back of the hand or the instep rose was proper, whereas a condition where this blood vessel did not rise at all was improper because of excess of tightening.

The exercise carried out for the arm during the tightening time was a type of exercise such that a dumbbell was lifted to contract the muscle of the arm and the dumbbell was then lowered to relax the muscle of the arm. On the other hand, the exercise carried out for the thigh during the tightening time was a squat type of exercise.

This muscle training was carried out for 30 minutes a day, and was repeated three times a week every other day for two weeks, i.e., totally six times for two weeks. After the two weeks, the muscles of the arm and the thigh of each man tightened with the tightening tools were compared with those not tightened with the tightening tools. As the result of comparison, the muscles subjected to the muscle training with use of the tightening tools were enlarged by 5%.

This result shows that the muscular strength was greatly increased in a short period of time by the muscle training according to the preceding method as compared with the conventional muscle training.

### Example II

Muscle training similar to that in Example I was carried out, with the exception that the tightening time was set within about 15 minutes and no exercise such as the lifting/lowering of a dumbbell and the squat type of exercise was carried out with the tightened condition maintained. After the two-week total training , the muscles of the arm and the thigh tightened were compared with those not tightened, and it was confirmed that the muscles subjected to the muscle training with use of the tightening tools were increased by 2%.

### Example III

With use of a tightening tool similar to that used in Example I, training for strengthening the muscle of the thigh was carried out as a bicycle sport being carried out.

Ten men aged from 19 to 32 were selected as a subject for the bicycle sport, and the tightening tool was applied to a thinner one of both thighs of each man at a position between a quadriceps muscle of thigh a biceps muscle of thigh and a greatest gluteal muscle.

The subject part was tightened by the tightening tool for about 30 minutes to block the flow of blood. In the event that the toes of the foot were numbed and lost sensation, the tightened condition was immediately released to end the muscle training.

This muscle training with the bicycle sport was carried out for 30 minutes a day, and was repeated three times a week every other day for two weeks, i.e. , totally six times for two weeks. After the two weeks, the muscle of the thigh of each man tightened by the tightening tool was compared with that not tightened by the tightening tool of each man. As the result of comparison, the muscle subjected to the muscle training with use of the tightening tool was enlarged by 5%.

This result shows that the muscular strength was greatly increased in a short period of time as compared with the conventional muscle training by incorporating the muscle training into the exercise as the bicycle sport without carrying out a special muscle training separately from an intended sport.

## Claims

1. A tightening tool for sports training comprising an elastic member (2) which is arranged to form a loop (L) of variable circumference for encircling a selected part of a limb, and locking means (3,6) for holding the loop formed from the flexible member during use at a desired circumference; characterised in that the tool comprises at least a detecting portion of indicating means (10) for detecting the force exerted by the loop on the encircled part of a limb, in use.

2. A tool according to claim 1, additionally comprising a lining (4), which, in use, is innermost along the circumference of the loop formed from the elastic member (2) .

3. A method for enlarging a muscle of a human being, the method comprising encircling a selected part of a limb with a loop of the tightening tool according to claim 1 or claim 2, and reducing the circumference of the loop to tighten the loop thereby restricting flow of blood to the muscle to be enlarged.

4. A method according to claim 3, wherein the reduced circumference of the loop is maintained while the muscle is exercised.

5. A method according to claim 4, wherein the exercise involves contracting the muscle isometrically or isotonically.

6. A method according to any one of claims 3 to 5, in which the reduced circumference of the loop is maintained for no greater than 30 minutes.

7. A method according to claim 6, wherein the reduced circumference of the loop is maintained for no greater than 15 minutes.

## Patentansprüche

1. Spanngerät für Sporttraining mit einem elastischen Bauteil (2), welches angeordnet ist, um eine Schleife (L) veränderlichen Umfangs für das Umschlingen eines ausgewählten Teils eines Körperglieds auszuformen, und einer Verriegelungseinrichtung (3, 6) für das Halten der Schleife, die durch das flexible Bauteil ausgeformt ist, während der Verwendung auf einem gewünschten Umfang,
***dadurch gekennzeichnet, daß***
das Gerät zumindest einen Erfassungsabschnitt einer Anzeigeeinrichtung (10) hat, für das Erfassen der Kraft, welche durch die Schleife auf den umschlungenen Teil eines Körperglieds während der Verwendung aufgebracht wird.

2. Gerät nach Anspruch 1, zusätzlich
***gekennzeichnet durch***
eine Verkleidung (4), die im Gebrauch sich am innersten Bereich entlang des Umfangs der Schleife erstreckend aus einem elastischen Bauteil (2) ausgeformt ist.

3. Verfahren zur Vergrößerung eines Muskels eines menschlichen Körpers, wobei das Verfahren umfaßt:
Umschlingen eines ausgewählten Teils eines Körperglieds mit einer Schleife des Spanngeräts gemäß Anspruch 1 oder Anspruch 2 und Verringern des Umfangs der Schleife, um die Schleife anzuspannen und hierdurch ein Blutfluß zu dem Muskel zu beschränken, der vergrößert werden soll.

4. Verfahren nach Anspruch 3,
***dadurch gekennzeichnet, daß***
der reduzierte Umfang der Schleife aufrechterhalten wird, während der Muskel bewegt wird.

5. Verfahren nach Anspruch 4,
***dadurch gekennzeichnet, daß***
die Bewegung eine isometrische oder isotonische Kontraktion des Muskels umfaßt.

6. Verfahren nach einem der Ansprüche 3 bis 5, wonach der reduzierte Umfang der Schleife für nicht mehr als 30 Minuten aufrechterhalten bleibt.

7. Verfahren nach Anspruch 6,
***dadurch gekennzeichnet, daß***
der reduzierte Umfang der Schleife für nicht länger als 15 Minuten aufrechterhalten bleibt.

## Revendications

1. Dispositif de serrage pour entraînement sportif comprenant un organe élastique (2) qui est agencé de manière à former une boucle (L) de circonférence variable pour entourer une partie sélectionnée d'un membre, et un moyen de blocage (3, 6) pour maintenir la boucle formée avec l'organe élastique à une circonférence souhaitée durant l'utilisation ; caractérisé en ce que le dispositif comprend au moins une partie de détection d'un moyen indicateur (10) pour détecter la force exercée par la boucle sur la partie entourée d'un membre, durant l'utilisation.

2. Dispositif selon la revendication 1, comprenant en outre un revêtement (4) qui, à l'utilisation, est la partie la plus interne le long de la circonférence de la boucle formée avec l'organe élastique (2).

3. Procédé pour développer un muscle d'un être humain, le procédé comprenant les opérations consistant à entourer une partie sélectionnée d'un membre avec une boucle du dispositif de serrage selon la revendication 1 ou la revendication 2, et réduire la circonférence de la boucle pour serrer la boucle de manière à restreindre l'afflux de sang jusqu'au muscle à développer.

4. Procédé selon la revendication 3, dans lequel la circonférence réduite de la boucle est conservée pendant toute la durée d'exercice du muscle.

5. Procédé selon la revendication 4, dans lequel l'exercice implique la contraction isométrique ou isotonique du muscle.

6. Procédé selon l'une quelconque des revendications 3 à 5, dans lequel la circonférence réduite de la boucle est conservée pendant une durée n'excédant pas 30 minutes.

7. Procédé selon la revendication 6, dans lequel la circonférence réduite de la bouche est conservée pendant une durée n'excédant pas 15 minutes.
